(19) Europäisches Patentamt
European Patent Office
Office européen des brevets

(11) EP 2 467 379 B1

(12) **EUROPEAN PATENT SPECIFICATION**

(45) Date of publication and mention of the grant of the patent:
**02.03.2016 Bulletin 2016/09**

(21) Application number: **10745177.5**

(22) Date of filing: **18.08.2010**

(51) Int Cl.:
***C07D 403/06*** *(2006.01)* ***A61K 31/506*** *(2006.01)*
***A61P 31/10*** *(2006.01)*

(86) International application number:
**PCT/EP2010/005072**

(87) International publication number:
**WO 2011/020605 (24.02.2011 Gazette 2011/08)**

(54) **Process for the production of coevaporates and complexes comprising voriconazole and cyclodextrin**

Verfahren zur Herstellung von Coevaporaten und Komplexen von Voriconazol und Cyclodextrin

Procédé pour la préparation des coévaporates et complexes comportant du voriconazole et cyclodextrine

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **19.08.2009 EP 09010681**
**11.01.2010 EP 10000183**

(43) Date of publication of application:
**27.06.2012 Bulletin 2012/26**

(73) Proprietor: **ratiopharm GmbH**
**89079 Ulm (DE)**

(72) Inventors:
- **SCHEIWE, Max, Werner**
**79689 Maulburg (DE)**
- **WINTER, Sven**
**59269 Neubeckum (DE)**
- **GRUN, Christoph**
**89077 Ulm (DE)**

(74) Representative: **Ter Meer Steinmeister & Partner Patentanwälte mbB**
**Nymphenburger Straße 4**
**80335 München (DE)**

(56) References cited:
**EP-A1- 2 018 866** **EP-B1- 0 440 372**
**CN-A- 1 813 751**

EP 2 467 379 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

The present invention relates to a process for producing a coevaporate comprising (a) voriconazole, (b) cyclodextrin, and (c) optionally an organic solvent, said process comprising the steps of (i) dissolving voriconazole and cyclodextrin in the organic solvent, and (ii) removing the solvent completely or partially. Furthermore, the present invention relates to the use of said coevaporate for the production of a voriconazole-cyclodextrin complex.

"Voriconazole" is the INN name of (2R,3S)-2-(2,4-difluorophenyl)- 3-(5-fluoropyrimidin-4-yl)-1-(1H-1,2,4-triazol-1-yl)butan-2-ol and is characterized by the following chemical formula:

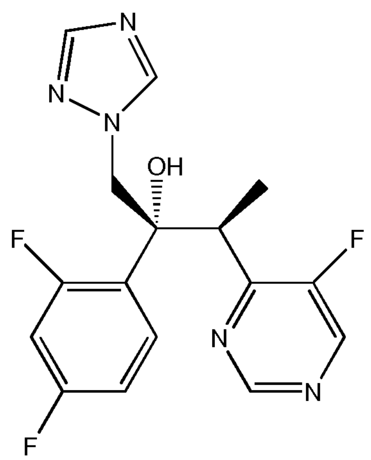

Voriconazole is used in the treatment of invasive aspergillosis, which may occur in immunocompromised patients, including allogeneic BMT, other hematologic cancers, and solid organ transplants. Voriconazole is further indicated for the treatment of candidemia, of invasive Candida infections and of fungal infections caused by Scedosporium spp. and Fusarium spp.. Voriconazole is marketed under the tradename VFEND in the form of a powder for solution for infusion. Since Voriconazole is a weak base and only slightly soluble in water, VFEND is provided in form of a powder for solution for infusion, wherein voriconazole is present in form of a cyclodextrin inclusion complex. Alternatively, voriconazole is marketed in form of film-coated tablets and in form of a powder for suspension. Due to the low solubility, micronisation of the API is recommendable.

The synthesis of voriconazole is described in EP 0 440 372 B1. A process for forming a voriconazole-cyclodextrin inclusion complex is disclosed in Example 17 of EP 0 440 372 B1. Further, voriconazole-cyclodextrin inclusion complexes are described in WO 91/11172, WO 98/58677 and EP 2 018 866 A1. However, all these prior art references disclose time-consuming methods for producing such inclusion complexes. For example, the method of EP 0 440 372 requires stirring for 2 days. The excessive time required by the slow dissolution of voriconazole is a critical process step and an unpredictable factor upon scale-up. Furthermore, it has been found that the reconstitution properties (properties when the solid voriconazole-cyclodextrin complexes are dissolved in water to give solutions suitable for infusion) are still enhancable.

EP 2 018 866 A1 discloses a process for improving the solubility of voriconazole in aqueous solution. Further EP 2 018 866 A1 describes a pharmaceutical composition comprising voriconazole and a beta-cyclodextrin, obtainable by this process, a method of treating a fungal infection and the use of a therapeutically effective amount of the composition comprising voriconazole and a beta-cyclodextrin and at least one pharmaceutically acceptable excipient for the preparation of a medicament for treating a fungal infection.

EP 0 440 372 B1 discloses specific triazole compounds, having antifungal activity. Further, the specific triazole compounds and their derivatives are described to be used in the treatment of fungal infections in animals and human beings.

CN 1 813 751 A relates to a voriconazole injection, wherein the injection includes 0.01-20 wt.% voriconazole, 0-60 wt.% cyclodextrin or cyclodextrin derivative, 0-75 wt.% water-soluble excipient, 0-20 wt.% solubilizer and injection water. Further, a method for preparing the voriconazole injection is described, wherein the method includes: a) mixing the above-mentioned raw materials, b) using active carbon to remove impurity and heat source, c) filtering, d) fine filtering, e) filling, f) sterilizing, g) checking and h) putting in storage.

Hence, it was an object of the present invention to overcome the drawbacks of the above-mentioned methods.

In particular, voriconazole should be provided in form of a cyclodextrin complex in solid form having superior reconstitution properties. Preferably, upon addition of water, a clear solution should be formed within less than one minute, preferably less than 30 seconds. Such superior reconstitution properties are desirable for the patient or for the

physician administering the resulting solution to the patient. Generally, superior reconstitution properties result in a better compliance.

Furthermore, it was an object of the invention to provide a superior method for producing voriconazole-cyclodextrin inclusion complexes. The method should be neither cost nor time consumptive. The method should be carried out in a large scale with an advantageous room/space yield.

In addition, voriconazole should be provided in a non-hygroscopic form having high solubility, high permeability and showing high storage stability.

Moreover, it was an object of the present invention to provide an oral dosage form having superior dissolution properties. Preferably, micronization of voriconazole should not be necessary.

The objects of the present invention can be solved by a coevaporate comprising

(a) voriconazole,
(b) cyclodextrin, and
(c) optionally an organic solvent, and its use in the production of voriconazole-cyclodextrin inclusion complexes.

Therefore, a subject of the present invention is a process for producing a coevaporate comprising

(a) voriconazole,
(b) cyclodextrin, and
(c) optionally an organic solvent,

said process comprising the steps of

(i) dissolving voriconazole and cyclodextrin in the organic solvent,
(ii) removing the solvent completely or partially.

A further subject of the present invention is a coevaporate, obtained by the above illustrated process.

Still a further subject of the present invention is a process for producing a voriconazole-cyclodextrin complex, preferably in solid form, said process comprises the steps of

(i) dissolving voriconazole and cyclodextrin in the organic solvent,
(ii) removing the solvent completely or partially in order to form a coevaporate,
(iii) dissolving the coevaporate in water, and
(iv) removing the water.

In addition, another subject of the present invention is a process for producing sterile vials containing a voriconazole-cyclodextrin complex in solid form, said process comprises the steps of

(i) dissolving voriconazole and cyclodextrin in the organic solvent,
(ii) removing the solvent completely or partially in order to form a coevaporate,
(iii) dissolving the coevaporate in water,
(iii-2) subjecting the resulting aqueous solution to a sterilisation step,
(iii-3) filling the sterilized solution, preferably under aseptic conditions, into vials,
(iv) removing the water by lyophilization, and
(v) sealing the vials.

The above illustrated subjects of the present invention are alternative solutions of the above outlined objectives.

The term "voriconazole" (a) as used in the present application refers to voriconazole in the form of the free base, as well as to its pharmaceutically acceptable salts (preferably derived from inorganic or organic acids), solvates, hydrates, enantiomers, polymorphs or mixtures thereof. Examples for pharmaceutically acceptable salts are acetate, adipate, alginate, citrate, aspartate, benzoate, benzenesulfonate, bisulfate, butyrate, camphorate, camphorsulfonate, digluconate, cyclopentanepropionate, dodecylsulfate, ethanesulfonate, glucoheptanoate, glycerophosphate, hemisulfate, heptanoate, hexanoate, fumarate, hydrochloride, hydrobromide, hydroiodide, 2-hydroxy-ethanesulfonate, lactate, maleate, mandelate, methansulfonate, nicotinate, 2-naphthalenesulfonate, oxalate, palmate, pectinate, persulfate, 2-phenylpropionate, picrate, pivalate, propionate, salicylate, succinate, sulfate, tartrate, thiocyanate, tosylate, mesylate, and undecanoate. Preferably, voriconazole is used in the form of the free base.

Besides voriconazole (a) the coevaporate comprises (b) cyclodextrin and (c) optionally an organic solvent.

The term "cyclodextrin" generally refers to non-reducing cyclic oligosaccharides. Preferably, said cyclic oligosaccharides comprise six, seven or eight glucose units linked by alpha-1,4 interglycosidic bonds. In the present invention cyclodextrins comprising seven glucopyranose units (= beta-cyclodextrins) are preferred.

Preferably, within this invention the beta-cyclodextrin is used as component (b). Beta-cyclodextrin is a ring-shaped molecule, made up of seven glucose units linked by alpha-1,4 bonds. More preferably, the beta-cyclodextrin is chemically modified, especially alkylated or hydroxyl-alkylated. Examples of suitable modified beta-cyclodextrins are dimethyl beta-cyclodextrin, hydroxypropyl betacyclodextrin, hydroxyethyl beta-cyclodextrin, dihydroxypropyl beta-cyclodextrin, glucosyl beta-cyclodextrin, diglucosyl beta-cyclodextrin, maltosyl beta-cyclodextrin, maltotriosyl beta-cyclodextrin and dimaltosyl beta-cyclodextrin, and mixtures thereof such as maltosyl beta-cyclodextrin/dimaltosyl beta-cyclodextrin.

In a preferred embodiment hydroxypropylated beta-cyclodextrin having the following structure is used:

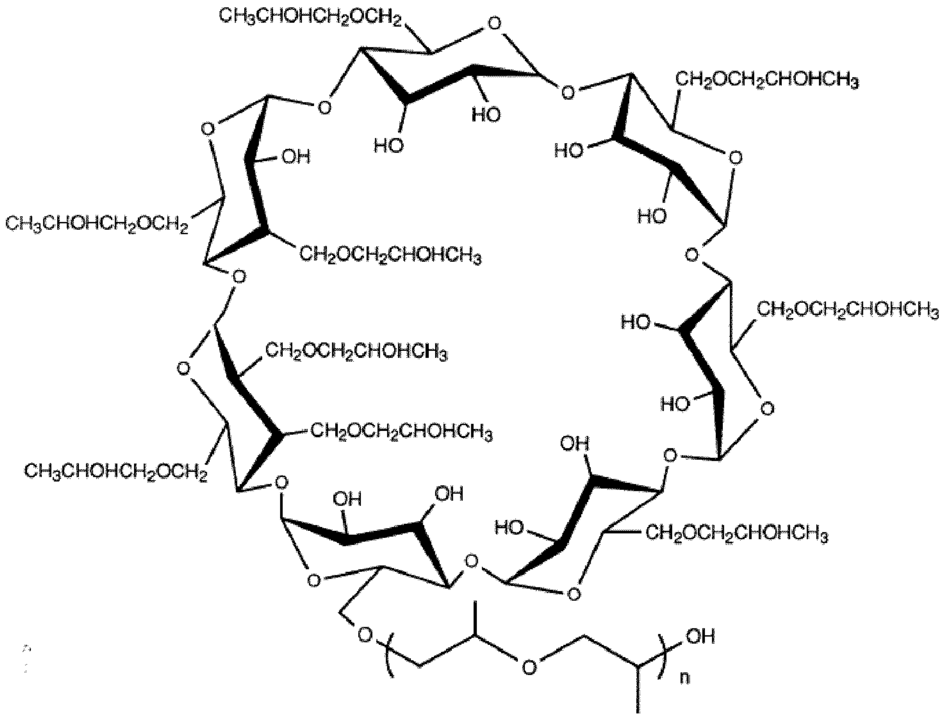

In case of hydroxypropylated beta-cyclodextrin, the average degree of substitution preferably varies from 3 to 6, more preferably from 4 to 5, in particular about 4.6. The average degree of substitution is understood as number of substituents per cyclodextrin ring. Especially an average degree of substitution of 4 to 5, in particular of about 4.6, leads to superior dissolution properties.

Moreover, preferably cyclodextrin having a bulk density of from 150 to 700 milligram/$cm^3$, more preferably from 200 to 350 milligram/$cm^3$ is used.

Furthermore, it is particularly preferred that in all embodiments of the present invention cyclodextrins are used in the form of cyclodextrin hydrate, particularly hydroxypropylated beta-cyclodextrin is used in the form of hydroxypropylated beta-cyclodextrin hydrate. In a preferred embodiment the water content of the cyclodextrin (b) is from 2 to 12 wt.-%, particularly from 8 to 10 wt.-%, based on the total weight of the cyclodextrin.

Generally, cyclodextrins form an inner cavity. Within this application said cavity is referred to as "nanocavity".

In the process for producing the coevaporate according to the present invention, the molar ratio of cyclodextrin (b) to voriconazole (a) usually is 1 : 2 to 5 : 1, preferably from 4 : 1 to 1 : 1, more preferably from 2.9 : 1 to 2.0 : 1. Thus, it is preferred that the voriconazole functions as "bifunctional guest" within the cyclodextrin cavity.

Besides voriconazole (a) and cyclodextrin (b) the coevaporate preferably comprises residual amounts of an organic solvent (c).

Generally, any organic solvent capable of dissolving voriconazole as well as cyclodextrin (at least partially but preferably completely) is suitable. Water is not regarded as organic solvent (c).

In a preferred embodiment an organic solvent having a relative permittivity (=$\varepsilon_r$) from 3 to 40, preferably from 4 to 38, more preferably from 5 to 35, still more preferably from 8 to 32, measured at 20 °C, is used.

The permittivity of a substance is a characteristic, which describes how it affects any electric field set up in it. A high permittivity tends to reduce any electric field present. The capacitance of a capacitor can be increased by increasing the permittivity of the dielectric material. The permittivity of free space (or a vacuum), $\varepsilon_0$, has a value of $8.9 \times 10^{-12}$ F

$m^{-1}$. The permittivity of a material is usually given relative to that of free space, it is known as relative permittivity, $\varepsilon_r$.

In Table 1 below the relative permittivity of several organic solvents (and water), measured at 20 °C, is given.

**Table 1:**

| **Solvent** | $\varepsilon_r$ | **Solvent** | $\varepsilon_r$ |
|---|---|---|---|
| Water | 80.0 | Pentane | 1.82 |
| Methanol | 32.6 | Petroleum ether | 1.9 |
| Ethanol | 25.8 | Hexane | 1.88 |
| Isopropanol | 26.0 | Heptane | 1.97 |
| Nitromethane | 39.4 | Cyclohexane | 2.06 |
| Acetonitrile | 38.8 | Decaline | 2.13 |
| Acetone | 21.5 | Toluol | 2.34 |
| n-propanol | 22.2 | m-xylene | 2.38 |
| n-butanol | 19.2 | Ethylbenzene | 2.42 |
| Butanone | 18.0 | o-xylene | 2.57 |
| Ethylene chloride | 10.13 | Tetralin | 2.66 |
| Methylene chloride | 9.08 | Dioxane | 3.0 |
| Methyl acetate | 7.08 | Triethylamine | 3.2 |
| Acetic acid | 6.29 | Diethyl ether | 4.34 |
| Ethyl acetate | 6.11 | | |
| Chloroform | 5.14 | | |

Generally, suitable organic solvents (c) might be selected from $C_3$-$C_6$ ketone, a $C_5$-$C_9$ aliphatic or aromatic hydrocarbon, optionally substituted e.g. with halogen, a $C_3$-$C_6$ ester, a $C_2$-$C_6$ alcohol, a $C_2$-$C_6$ ether, DMAc, DMF, DMSO, NMP and mixtures thereof.

In a preferred embodiment the organic solvent (c) is an alcohol, preferably a $C_2$-$C_6$ alcohol, still more preferably ethanol and isopropyl alcohol. Ethanol is particularly preferred.

Generally, the organic solvent (c) can comprise mixtures of two or more of the above mentioned solvents.

The process for producing the coevaporate according to the present invention comprises the steps of

(i) dissolving voriconazole and cyclodextrin in the organic solvent,
(ii) removing the solvent completely or partially.

In step (i) voriconazole (a) and cyclodextrin (b) are dissolved, preferably completely dissolved, in the organic solvent (c). Dissolution can be carried out at room temperature, preferably temperatures from 10 to 40 °C, more preferably from 15 to 30 °C, are used. Usually, dissolution can be achieved even without stirring.

The amount of organic solvent applied in step (i) for dissolving voriconazole and cyclodextrin usually influences the process with regard to process time and process costs. In a preferred embodiment voriconazole is dissolved in an amount of 10 to 100 mg per ml organic solvent, more preferably of 20 to 80 mg/ml, still more preferably from 30 to 60 mg/ml, most preferably from 35 to 45 mg/ml.

In a preferred embodiment cyclodextrin is dissolved in an amount of 100 to 1100 mg per ml organic solvent, more preferably of 200 to 850 mg/ml, still more preferably from 300 to 650 mg/ml, most preferably from 350 to 480 mg/ml.

In step (ii) the organic solvent (c) is completely or partially removed. It is preferred that the solvent is partially removed, resulting in an amount of solvent (c) in the coevaporate. In a preferred embodiment the coevaporate comprises an amount of residual organic solvent (c) of 0.1 to 10 wt.%, more preferably of 0.5 to 5 wt.%, still more preferably of 0.6 to 4 wt.%, particularly preferably of 0.7 to 3.0 wt.%, based on the total weight of the coevaporate.

In a further preferred embodiment, starting compounds and process conditions are chosen such that the resulting coevaporate comprises

(a) 0.1 to 30 wt.%, preferably 1 to 20 wt.%, more preferably 5 to 15 wt.% voriconazole,

(b) 70 to 99.9 wt.%, preferably 80 to 99 wt.%, more preferably 85 to 95 wt.% cyclodextrin, and
(c) 0 to 20 wt.%, preferably 0.1 to 10 wt.%, more preferably 0.5 to 5 wt.% organic solvent,

based on the total weight of the coevaporate.

In step (ii) the organic solvent (c) can be removed under elevated temperature and/or under reduced pressure. In a preferred embodiment the solvent is removed at a temperature between 30 and 90 °C, preferably between 40 and 80 °C, more preferably between 50 and 70 °C, in particular, at about 60 °C. The solvent can be removed at a pressure from 0.1 to 1000 mbar, preferably from 1 to 200 mbar, more preferably from 5 to 100 mbar, still more preferably from 10 to 50 mbar, in particular from 30 to 40 mbar. At laboratory scale the duration of step (ii) might range from 0.5 to 2.0 hours, preferably about 90 minutes. The removal of the organic solvent can be, for example, carried out in a vacuum rotary evaporatory, e.g. a Büchi® Rotavapor.

It has been unexpectedly found that the content of TAP impurities can be lowered if specific evaporation conditions are chosen. The term "TAP" hereinafter refers to (1-(2,4-difluorophenyl)-2-(1 H-1,2,4-triazol-1-yl) ethanone), as shown by the chemical formula below.

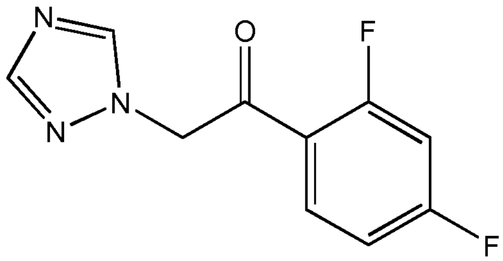

Preferably, the evaporation conditions are chosen such that the TAP content in the coevaporate is less than 0.20 wt.-%, more preferably less than 0.15 wt.-%, still more preferably less than 0.10 wt.-%. In a preferred embodiment the solvent is removed at a temperature between 30 °C and 70 °C, preferably between 35 °C and 65 °C, more preferably between 40 °C and 60 °C. Depending on the batch size and size and filling volume of the evaporation flask the evaporation time might take up to four hours. Furthermore, in a preferred embodiment the removing is carried out within 10 to 180 minutes, more preferably within 15 and 120 minutes, still more preferably within 20 and 80 minutes, most preferably within 25 and 70 minutes.

In a preferred embodiment the resulting coevaporate is provided in form of a glassy material, preferably in form of a thermodynamically activated, solid, lacquer-like, white or semi-transparent (milky-white) and/or glassy material.

Within this application the residual amount of solvent (c) (in the coevaporate and in the voriconazole-cyclodextrin inclusion complex) is determined via gas chromatography. Conditions as outlined below were chosen.

| | |
|---|---|
| *Apparatus:* | |
| Gas chromatograph: | Shimadzu GC-17A |
| Detector: | Flame ionization detector (FID) |
| Injector: | Shimadzu AOC-5000 auto injector |
| *Software:* | Shimadzu Class-VP 7.4 Version |
| *Gases:* | |
| Carrier gas: | Helium (99.999 %) |
| Other gases: | Nitrogen (99.999%) |
| | Synthetic air (99.999%) |
| | Hydrogen (from Whatman Hydrogen generator) |

*Column:* Rtx624 (30 m × 0.32 mm × 1.8 mm) (Restek)

TEMPERATURE PROGRAM:

| Rate (°C/min) | Temperature (°C): | Time (min): |
|---|---|---|
| - | 40 | 5.0 |
| 40 | 200 | 1.0 |

| | |
|---|---|
| Injector temperature: | 220 °C |
| Detector temperature: | 220 °C |
| Split ratio: | 30:1 |
| Velocity: | 30 cm/s |

Injection Program:

After incubation for 20 min at 90 °C, 1 ml sample of the vapor is injected into the gas chromatograph with a syringe of 95 °C.

Blank sample: 2 ml distilled water is measured in the vial and crimp capped.

Preparation of internal standard solution:

20 mg of methanol is measured into 10 ml graduated glass flask and made up to the mark with distilled water

Solutions for calibration:

Different amounts of ethanol (10, 25, 50, 75 and 100 mg) and 20 mg of methanol are accurately weighed into 10 ml graduated glass flasks and made up to the mark with distilled water. 100 ml of these solutions and 2 ml of distilled water are measured into HS vials (19.5 ml).

The calibration solution No. 3 is used to determine system suitability (sample SST). Requirement: RSD < 5% (RSD calculated for ethanol and methanol peak area ratio values)

Sample preparation:

100 mg of the sample is measured into the head space vial (19.5 ml) with 2 ml of distilled water and 100 ml of the internal standard solution.
The vials are dryed at 90 °C for 2 hours before sample preparation.
The ethanol concentration in the sample is calculated as follows:

$$c_{EtOH}(\mu g/g) = \frac{c_{MeOH}(\mu g/vial)a\frac{A_{EtOH}}{A_{MeOH}}}{m_{sample}(g/vial)}$$

| | |
|---|---|
| $c_{EtOH}$: | ethanol concentration in the sample |
| $c_{MeOH}$: | methanol concentration in the sample |
| $m_{Sample}$: | the mass of sample (measured in vial) |
| a: | coefficient of the linear internal standard calibration curve, determined by Shimadzu Class-VP 7.4 software |
| $A_{EtOH}$, $A_{MeOH}$: | the peak areas of ethanol and methanol (internal standard). |

The process of the present invention as illustrated above results in coevaporates comprising (a) voriconazole, (b) cyclodextrin, and (c) preferably residual amounts of organic solvent. The resulting coevaporates also are subjects of the present invention. Hence, a subject of the present invention are coevaporates obtained by a process as illustrated above. Regarding said coevaporates, all comments made above regarding preferred embodiments (e.g. regarding kind and amount of organic solvent, kind and amount of voriconazole and kind and amount of cyclodextrin) apply.

The resulting coevaporate can be regarded as an "intermediate" in the preparation of pharmaceutical formulations. In a preferred embodiment the coevaporate is further processed to give a stable voriconazole-cyclodextrin complex, preferably a voriconazole-cyclodextrin inclusion complex, preferably in solid form.

Therefore, in a further aspect the present invention relates to a process for producing a voriconazole-cyclodextrin complex, preferably in solid form, said process comprises the steps of

(i) dissolving voriconazole and cyclodextrin in the organic solvent,
(ii) removing the solvent completely or partially in order to form a coevaporate,

(iii) dissolving the coevaporate in water, and
(iv) removing water.

Steps (i) and (ii) refer to the process for producing the coevaporate. Essentially, all comments given above regarding preferred embodiments of the coevaporate apply.
In step (iii) the coevaporate is dissolved in water, preferably completely dissolved. In a preferred embodiment purified water (aqua purificata according to Ph.Eur 6.0/0008), highly purified water (aqua valde purificata according to Ph.Eur. 6.0/1927) or water for injection (aqua ad iniectabilia according to Ph.Eur. 6.0/0169) is used. Water for injection is particularly preferred. Furthermore, in step (iii) the term "water" also comprises aqueous solutions, e.g. saline.
The dissolution step can be carried out at room temperature or alternatively at temperatures e.g. between 5 °C and 50 °C. Usually, dissolution of the coevaporate occurs quite quickly, e.g. between 1 and 10 minutes. The dissolution speed can be increased by stirring.
The amount of water applied in step (iii) for dissolving the coevaporate usually influences the process with regard to process time and process costs. In an embodiment in step (iii) water is added in an amount, such that voriconazole is present in a concentration of 11 to 60 mg per ml water, preferably 12 to 50 mg/ml, more preferably 15 to 35 mg/ml, most preferably 18 to 25 mg/ml. Furthermore, in step (iii) water is added in an amount, such that cyclodextrin is present in a concentration of 110 to 600 mg per ml water, preferably 120 to 500 mg/ml, more preferably 150 to 350 mg/ml, most preferably 180 to 250 mg/ml.
It has been found that the amount of water applied in step (iii) for dissolving the coevaporate influences the residual organic solvent content of the resulting voriconazole-cyclodextrin complex as obtained in process step (iv) or (v), preferably of the resulting lyophilisate. Usually, water is added in an amount such that the residual organic solvent contents of the resulting voriconazole-cyclodextrin complex as obtained in process step (iv) or (v), preferably of the resulting lyophilisate, is less than 5000 ppm, in particular if ethanol is used as solvent. In a preferred embodiment in step (iii) water is added in an amount such that voriconazole is present in a concentration of 5 to 30 mg per ml water, preferably 10 to 25 mg/ml, more preferably 11 to 20 mg/ml, most preferably 12 to 16 mg/ml. It has been further found that a specific osmolality of the solution (iii) is desirable. In a preferred embodiment in step (iii) water is added in an amount such that the osmolality of the resulting solution (iii) ranges from 150 to 800 mosmol/kg, more preferably from 200 to 600 mosmol/kg, still more preferably from 225 to 500 mosmol/kg, most preferably from 250 to 450 mosmol/kg. The osmolality is defined and determined according to Ph.Eur. 6.0, 2.2.35.
Furthermore, it has been unexpectedly found that the amount of residual solvent in the resulting voriconazole-cyclodextrin complex as obtained in process step (iv) or (v), preferably of the resulting lyophilisate, can be reduced if step (iii) is carried out twice. Therefore, in a preferred embodiment step (iii) of the process of the present invention comprises the following sub-steps:

(iii-a) dissolving the coevaporate in water;
(iii-b) removing the water, preferably by evaporation; and
(iii-c) dissolving the evaporate, resulting from step (iii-b), in water.

Also for step (iii-a) the explanations as given above for step (iii) apply. In step (iii-b) the water is removed, preferably by evaporation. Preferably, the conditions are chosen such that the resulting evaporate has a water content of less than 20 wt.-%, more preferably less than 10 wt.-%, in particular 0.01 to 5 wt.-%. Again, for step (iii-c) the explanations as given above for step (iii) apply.
In a further preferred embodiment the solution resulting in step (iii) (or if the sub-steps are applied resulting in step (iii-a) or preferably in step (iii-c)) is subjected to a sterilisation step (= step iii-2). The sterilisation step (iii-2) usually can be carried out with methods common in the art. Preferably, the sterilisation step (iii-2) is carried out by sterile filtration. That means that the solution resulting in step (iii) preferably is sterilized by filtration through filters that remove bacteria and having a pore size of 0,22 $\mu$m. The membrane filters generally can be made from materials common in the art. Preferably, the membrane filters are made from nylon (e.g Aerodisc®, Ultipor® $N_{66}$, $N_{66}$ Posidyne®), cellulose derivatives like cellulose acetate or cellulose nitrate (e.g. a Nalgene® Filter Unit), mixed cellulose ethers, polyethersulfones, polysulfones, PTFE (e.g. Polydisc® TF) or PVDF. Most preferably nylon is used.
Consequently, it is preferred that step (iii) of the present invention comprises the following sub-steps: (iii-a), (iii-b), (iii-c) and (iii-2).
In step (iv) of the process of the present invention water is removed. Generally, the methods known in the art for removing solvents or water are suitable. Preferably, the solvent is removed by lyophilization (freeze-drying) or spray-drying. The removal of the solvent by lyophilization is particularly preferred, espcially, if the voriconazol-cyclodextrin complex should be provided in vials under aseptic conditions.
Alternatively, spray-drying can preferably be used. Spray-drying is particularly preferred if the resulting voriconazol-cyclodextrin complex should be provided in form of an oral dosage form. The spray-drying can be performed with

or without additives. As additives e.g. sorbitol, xylitol, polyethyleneglycol, dextrose or lactose can be used.

Generally, spray-drying can be carried out, using an inlet temperature of 120 to 220 °C, preferably about 180 °C, and an outlet temperature of about 70 to 120 °C, preferably of about 95 °C. For example, spray-drying can be carried out by using a Büchi® Lab Niro spray-drier.

Generally, the lyophilization process might comprise three stages:

Stage 1: Freezing the solution resulting from step (iii), step (iii-2) or preferably step (iii-3).

Stage 2: Main drying phase, where main parts of the solvent is removed at low temperatures and reduced pressure. Preferably, the pressure is reduced below the triple point of the solution resulting from step (iii) or step (iii-3).

Stage 3: Final drying phase, where bound water is removed. The temperature is raised preferably to the sublimation curve, in order to allow latent heat of sublimation.

In a preferred embodiment, stage 1 is carried out at temperatures between - 35 °C and - 60 °C, more preferably between - 40 °C and - 52 °C. The preferred freezing rate is from 0.1 to 5 K/min, more preferably from 0.5 to 2.0 K/min.

In a preferred embodiment stage 2 is carried out at temperatures between -25 °C and 0 °C, at pressures from 0.01 to 1 mbar. The preferred heating rate is from 0.05 to 5 K/min, more preferably from 0.2 to 1.0 K/min. Usually, the main drying stage ranges from 20 to 40 hours.

In a preferred embodiment stage 3 is carried out at temperatures between 10 °C and 45 °C at pressures from 0.001 mbar to 0.1 mbar. Usually, the final drying stage ranges from 20 to 40 hours.

After the water has been removed, the voriconazole-cyclodextrin complexes of the present invention are obtained, preferably in solid form. The fastest dissolving form of the voriconazole-cyclodextrin complexes of the present invention usually is achieved, if in step (iv) the solvent is removed by lyophilization.

The voriconazole-cyclodextrin complex as obtained by the present invention can be regarded as inclusion complex, preferably as "genuine" voriconazole-cyclodextrin inclusion complex. The term "genuine" indicates that the entire and complete amount of voriconazole is entrapped intercalated into the nanocavities of the cyclodextrin, i.e. voriconazole essentially is only present in intercalated form, that means essentially no adsorbed, un-entrapped crystalline voriconazole occurs. The formation of the genuine inclusion complex generally leads to a glassy solid form of voriconazole. Hence, the voriconazole-cyclodextrin inclusion complexes of the present invention preferably are essentially free of "solid" voriconazole, i.e. essentially free of crystalline voriconazole. The term "essentially" free means that the inclusion complexes of the present invention do not contain significant amounts of crystalline voriconazole, bound to the surface of complex particles. Preferably, the voriconazole inclusion complexes of the present invention comprise less than 5 wt.%, more preferably less than 2 wt.-%, more preferably less than 0.5 wt-% voriconazole in crystalline form, based on the total weight of the inclusion complex.

The completeness of the inclusion process can be monitored via solid state Raman microscopy/spectroscopy and/or by the SEM-ESD electron-microscopic surface mapping. Preferably, all inclusion complexes of the present invention are non-covalent inclusion complexes. Furthermore, preferably all inclusion complexes of the present invention are supramolecular inclusion complexes. In particular, all inclusion complexes of the present invention are non-covalent and supramolecular inclusion complexes. The term "supramolecular" is understood as describing self-organizing molecular interactions that result in the formation of new structures that stay together without establishing a covalent linkage.

Generally, the process of the present invention is suitable for preparing the inclusion complexes of the present invention, preferably achieving a yield of from 80 to 99 %, more preferably from 90 to 98 %.

In a preferred embodiment the residual water content of the inclusion complexes of the present invention is 0.01 to 5 wt.%, more preferably 0.1 to 3 wt.%, still more preferably 0.3 to 2.0 wt.%, most preferably 0.5 to 1.5 wt.%, based on the total weight of the complex. Preferably, the water content of the complexes of the present invention does not increase by more than 6 % (in particular, not more than by 3 %) during a storage period of 3 months, at a temperature of 25 °C and a humidity of 60 %.

Furthermore, the voriconazole-cyclodextrin complexes of the present invention usually are provided in a solid, form, having a bulk density of 150 to 400 $mg/cm^3$, preferably of 200 to 300 $mg/cm^3$, more preferably of 220 to 260 $mg/cm^3$. The voriconazole-cyclodextrin complexes preferably possess Hausner ratios in the range of 1.01 to 1.6, preferably of 1.06 to 1.4, more preferably between 1.08 to 1.3. The Hausner ratio is the ratio of tapped density to bulk density.

Bulk density and tapped density are determined according to USP 24, Test 616 "Bulk Density and Tapped Density".

The process of the present invention as illustrated above results in voriconazole-cyclodextrin complexes. The resulting complexes also are subjects of the present invention. Hence, a subject of the present invention are voriconazole-cyclodextrin complexes obtained by a process as illustrated above. Regarding said complexes, all comments made above regarding preferred embodiments (e.g. regarding kind and amount of organic solvent, kind and amount of voriconazole and kind and amount of cyclodextrin, particle size, bulk density etc., Hausner ratio, residual water content, residual solvent content etc.) apply.

Therefore, a voriconazole-cyclodextrin complex is preferably in solid form and comprises voriconazole, cyclodextrin, residual water and residual solvent, wherein the residual water content is from 0.1 to 3 wt.%, preferably from 0.5 to 1.8 wt.% and the residual solvent content is from 0.1 to less than 0.5 wt.%, preferably from 0.2 to 0.4 wt.%.

The residual water content is determined according to the Karl Fischer method as described in Ph. Eur. 6. edition, 2008, section 2.5.12.. The determination is done by coulometry, whereby a coulometer is used at 160 °C, preferably a Metrohm 831 KF Coulometer including a titration cell without diaphragm. Usually, a sample of 300 mg voriconazole-cyclodextrin complex is analyzed.

In this aspect, all comments made above regarding preferred embodiments (e.g. regarding kind and amount of organic solvent, kind and amount of voriconazole and kind and amount of cyclodextrin, particle size, bulk density etc., Hausner ratio, residual water content, residual solvent content etc.) apply as well.

The inclusion complexes (and optionally also the coevaporates) of the present invention can be applied in the form of pharmaceutical formulations.

In a preferred embodiment the pharmaceutical formulation is provided in form of a sterile vial, containing the inclusion complex of the present invention, wherein the inclusion complex can be redissolved, preferably with water for injection (aqua ad iniectabilia according to Ph.Eur. 6.0/0169), to give a solution for injection.

Therefore, a further subject of the present invention is a process for producing sterile vials containing a voriconazole-cyclodextrin complex in solid form, said process comprising the steps of

(i) dissolving voriconazole and cyclodextrin in the organic solvent,
(ii) removing the solvent completely or partially in order to form a coevaporate.
(iii) dissolving the coevaporate in water,
(iii-2) subjecting the resulting aqueous solution to a sterilisation step,
(iii-3) filling the sterilized solution, preferably under aseptic conditions, into vials,
(iv) removing water by lyophilization, and
(v) sealing the vials.

Steps (i) to (iii-2) and (iv) have been illustrated above. Steps (iii-3) and (v) can be carried out by methods known in the art. As sealing material rubbers (e.g. bromobutly or chlorobutyl rubbers) can be used, which may be coated with PTFE or siliconized. Preferably, siliconized chlorobutyl rubber is used.

The inventors of the present invention have found that the voriconazole-cyclodextrin complexes of the present invention have unexpected superior "reconstitution-properties". The term "reconstitution-properties" refers to the characteristics (e.g. dissolution time, appearance of the resulting solution, etc.) when the complexes of the present invention are redissolved, perferably in an aqueous medium, which is suitable for injection. For redissolving, water for injection is preferably used.

In another preferred embodiment, an aqueous medium is used, wherein the medium is chosen such that the osmolality of the resulting dissolution is from 100 to 300 mosmol/kg, preferably from 200 bis 290 mosmol/kg. Osmolality is a measure of the osmoles of solute per kilogram of solvent. Within this application, the term "osmolality" is defined and determined as disclosed in Ph. Eur., 6th edition, 2008, section 2.2.35.

Optionally, the osmolality is adjusted by using suitable redissolving agents as saline solutions, preferably having a sodium chloride content of 0.7 to 1.0 wt.% or a glucose solution, having a glucose content of 3 to 6 wt.%.

In an alternative embodiment the pharmaceutical formulation is provided in form of an oral dosage form, e.g. tablets, capsules, granulates.

Hence, a further subject of the present invention is an oral dosage form, comprising

(a) the coevaporate according to the present invention or more preferably the voriconazole-cyclodextrin complex according to the present invention; and
(b) pharmaceutical acceptable excipients.

Preferably, the oral dosage form is provided in form of tablets, more preferably film-coated tablets. The tablets preferably are prepared by direct-compression.

In the pharmaceutical formulation of the present invention one or more pharmaceutically acceptable excipient(s), such as fillers, binding agents, lubricants, glidants, anti-sticking agents and disintegrating agents, can be employed. Regarding the above-mentioned pharmaceutically acceptable excipients, the application refers to "Lexikon der Hilfsstoffe für Pharmazie, Kosmetik und angrenzende Gebiete", edited by H. P. Fiedler, 4th Edition, Edito Cantor, Aulendorf and earlier editions, and "Handbook of Pharmaceutical Excipients", third edition, edited by Arthur H. Kibbe, American Pharmaceutical Association, Washington, USA, and Pharmaceutical Press, London.

Finally, an organic solvent, having a relative permittivity from 3 to 40, measured at 20 °C, can be used for the production of a voriconazole-cyclodextrin inclusion complex. Regarding preferred embodiments of this aspect (e.g. permittivity, amounts and kinds of cyclodextrin etc.), it is referred to the illustrations given above.

The present invention is illustrated by the following examples.

## EXAMPLES

### Example 1: Preparation of a Voriconazole-Cyclodextrin Complex

3.0 g micronized voriconazole (= API) was weighed into a round bottom flask. 34.2 g solid hydroxypropylated beta-cyclodextrin (= HPBCD) having an average degree of substitution of 4.6 was added to the API (equivalent with 32.6 g HPBCD on the dry basis). The powder mixture was dissolved in ethanol (96 %). The ingredients were readily wetted and dissolved in ethanol at ambient temperature; the resulting liquid was viscous and clear. The solution was then dried in a vacuum rotary evaporator (Büchi® Rotavapor R-134). The bath of the equipment was set to T= 60 °C, the evaporation was performed at reduced pressure (p=32 mbar), until reaching constant weight (in duration of 90 minutes). The ethanol content of the coevaporate slightly exceeded 0.5%. The coevaporate was obtained in the form of a white or semi-transparent, glassy lacquer-like layer.

The coevaporate was dissolved in 150 ml purified water at ambient temperature. The obtained solution was then filtered through a disposable Nalgene® Filter Unit, equipped with a 0.22 μm cellulose nitrate filter. Based on in-process voriconazole content determination, a calculation was made resulting that 11.75 ml solution contains 200 mg API. Therefore, this value was established as filling volume. The filled vials were closed by rubber stoppers, their content was freezed in ethanol, cooled with dry ice. Then the rubber stoppers were removed and the content of the vials were freeze-dried for 24 hours at (-50) - (-52) °C chamber temperature and at 45-65 mTorr (6.0-8.7 Pa) pressure. Having the bulk solvent removed, the vials were kept for further 30 hours in the freeze-drier, but the chamber temperature was elevated to (+36) - (+37) °C (the pressure was unchanged). Finally, the vials were closed by chlorobutyl lyophilization stoppers, fastened manually with aluminum flip-off caps.

### Example 2: Preparation of a Voriconazole-Cyclodextrin Complex

Example 1 was repeated, wherein unmicronized voriconazole was used.

### Example 3: Preparation of a Voriconazole-Cyclodextrin Complex

Example 1 was repeated, wherein unmicronized voriconazole and hydroxypropylated beta-cyclodextrin (= HPBCD), having an average degree of substitution of 4.0, was used.

### Example 4: X-Ray Powder Diffraction

The X-ray diffractograms obtained for voriconazole and three voriconazole-cyclodextrin complexes prepared according to Examples 1 to 3 are shown in **Figure 1.**

The X-powder diffraction investigations were performed by using standard normal $CuK_{alpha}$ radiation. The reflection peaks were registered in 2-theta angle range of 5-40 degrees.

### Example 5: Differential Scanning Calorimetry (DSC)

Verifying data of a genuine complex formation was obtained by DSC.

**Figure 2** shows the DSC curves obtained for the plain API, its HPBCD-complexes and also the thermogram of the physical mixture composed of the constituents mixed in the ratio equivalent with that of the optimized complex. The intense endothermic peak (at 132.1 °C), corresponding to the melting enthalpy of voriconazole (appearing somewhat distorted studying the physical mixture), completely disappears when complexed with HPBCD. This phenomenon indicates that the intermolecular forces stabilizing the crystalline structure of the plain drug no longer exist in the complex, i. e. the API is in a so-called 'molecularly dispersed' state. These findings undoubtedly indicate that in the process of the present invention HPBCD forms an inclusion complex with voriconazole.

Differential scanning calorimetric measurements were performed by using a Perkin Elmer 2 instrument, applying the following experimental parameters:

- sample weight: 3.5-7.5 mg
- atmosphere: Ar (99.999%)

- heating range: 10 K/min (5 K/min was also tested and showed comparable results)
- sample holder: A1 (the cap was not crimped, but only placed on the bottom part)
- calibration was performed by using indium standard
- temperature range: 320-480 K

**Example 6: Compositional Characteristics**

The compositional characteristics of the complexes of the present invention according to Examples 1 to 3 are given below in Table 2:

**Table 2**

| voriconazole/ HPBCD complex | Appearance in solid state | Voriconazole content in solid complex* | HPBCD content in solid complex* | βCD content in solid complex* | Residual water content | Residual ethanol content |
|---|---|---|---|---|---|---|
| Example 3 | lyophilizate | 8.47 % | 89.2 % | 0.66 % | 1.3 % | 0.24 % |
| Example 1 | lyophilizate | 8.37 % | 90.0 % | 0.55 % | 1.1 % | 0.32 % |
| Example 2 | lyophilizate | 8.27 % | 91.2 % | 0.57 % | 1.1 % | 0.27 % |
| * Value corresponds to HPBCD content as it is (e.g. not corrected with water content) | | | | | | |

The results indicate that when applying the preparation process of the present invention, superior batch-to-batch compositional reproducibility could be assured.

The determination of the HPBCD and beta-cyclodextrin (BCD) content was done by HPLC. A detailed description of the chromatographic conditions is given below.

Apparatus:

Agilent 1100 Series Quaternary Pumping System
Agilent 1100 Series Degasser
Agilent 1100 Series Thermostatted Column Compartment
Agilent 1100 Series Thermostatted Autosampler
Agilent 1200 Series Diode Array Detector
Alltech 3300 Evaporative Light Scattering Detector (ELSD2)
Agilent ChemStation for LC 3D, Version No: B.02.01-SR[260]

Column:

CD-Screen, 5 μm, 4.0 x 250 mm (ChiroQuest Ltd, Budapest, Hungary)
Code: CDS2

Mobile phase:

*Channel A:*

| | |
|---|---|
| Water | 1000 ml |

*Channel A:*

| | |
|---|---|
| Methanol | 900 ml |
| Water | 100 ml |

Gradient Program:

| Time (min) | B Channel %: | Flow rate (ml/min) |
|---|---|---|
| 0 | 45 | 0.8 |
| 5 | 45 | 0.8 |
| 9 | 100 | 0.8 |

| | |
|---|---|
| Stop time: | 16 min |
| Post time: | 8 min |
| Column temperature: | 15°C |
| Injected volume: | 2 µl for HPBCD assay |
| | 50 µl for BCD and impurities |

Detection:

*DAD:* 200 nm (bandwidth 4 nm, reference 360 nm, bandwidth: 100 nm)

*ELSD parameters:*

| | |
|---|---|
| Drift tube temperature: | 70 °C |
| Gas flow | 1.51/min |
| Gain: | 1 |

Heart cutting valve off: 7.5 - 8.5 for BCD and impurities

Sample preparation:

Solvent:

Prepare a solvent consisting of 50 % MeOH and 50 % purified water.

BCD Calibration Solutions:

Approximately 10 - 20 mg BCD standard (3 parallels) were weighed accurately and transferred into 5 ml volumetric flasks. The substances were dissolved and diluted to the volume with the solvent (*BCD stock solutions*). The calibration solutions in the range of 0.06 to 0.3 mg/ml were prepared with appropriate dilution of the *BCD stock solutions* with the solvent. One of the calibration solutions (conc. 0.5 mg/ml) was used for system suitability test (5 parallel injections).

HPBCD Calibration Solutions:

Approximately 75 - 225 mg HPBCD standard (5 parallels) were weighed accurately and transferred into 5 ml volumetric flasks. The substances were dissolved and diluted to volume with the solvent.

Sample Solutions:

Approximately 150 mg HPBCD sample were weighed accurately and transferred into 5 ml volumetric flasks. The substances were dissolved and diluted to volume with the solvent.

Calculation:

The HPBCD and BCD contents were calculated with the Agilent ChemStation Software by external standard method with the HPBCD and BCD calibration curve, respectively. The signal of HPBCD was integrated as one peak. The calibration curves were calculated with the Agilent ChemStation Software by "Power" curve type (origin: ignore,

weight: equal).

The concentration of the voriconazole solutions were determined by UV/VIS spectrophotometry based on absorbance peak found at wavelength 256 nm.

A linear calibration curve was fitted to the experimental absorbance values (**Figure 3**).

### Example 7: Physico-Chemical Characteristics

The physico-chemical characteristics of the complexes of the present invention according to Examples 1 to 3 are given below in Table 3:

**Table 3:**

| voriconazole / HPBCD complex | Appearance in solid state | Bulk density | Amorphous -ness / crystallinity | pH |
|---|---|---|---|---|
| Example 3 | lyophilizate | 0.24 g/cm$^3$ | amorphous | with water for injection: **6.2** with physiologic salt solution.: **6.4** |
| Example 1 | lyophilizate | 0.24 g/cm$^3$ | amorphous | with water for injection: **6.4** with physiologic salt solution.: **6.6** |
| Example 2 | lyophilizate | 0.24 g/cm$^3$ | amorphous | with water for injection: **6.4** with physiologic salt solution.: **6.6** |

Tables 3 and 4 clearly indicate that superior characteristics are obtained for all examples. That means, superior characterstics can be obtained, irrespective whether the voriconazole used as starting material was micronized or not.

### Comparative-Example 8: Example 17 of EP 0 440 372

Example 17 EP 0 440 372 was reworked with HPBCD.
The specified process was especially time-consuming.

The resulting "solution" obtained remained slightly hazy.

As a conclusion, in terms of time consumption and microbial considerations the process of the present invention offers a more controlled and rapid preparation method. Since complex formation takes place in a single phase, the process of the present invention is highly robust regarding API particle size as well.

### Comparative-Example 9: Example 1 of EP 1 001 813

Example 1 of EP 1 001 813 was reworked. The ingredients were weighed in the ratio specified in the patent application document (regarding quantity, total volume of 65 ml was applied to obtain representative sample). First, sulphobutylether beta-cyclodextrin (SBECD) was added to water for injections (which readily dissolved). Having voriconazole added to the solution, the mixture was vigorously stirred. The suspension also had to be homogenized by hand a couple of times (to remove the particles stuck to the wall of the vessel). The quantitative dissolution of the particles was reached at ambient temperature after one and a half hours. This rate was found surprisingly low, suggesting thus a highly difficult scale-up prospective.

The obtained solution was filtered through a membrane filter of 0.22 mm pore size, and lyophilized. A unit (comprising 200 milligram voriconazole and 3.2 g SBECD) was reconstituted with water for injection. The complex redissolved within one minute, but having the material dissolved, air microbubbles were still present in the solution for further two minutes. Therefore, total time of approximately three minutes was required until the administrable state of the formulation was obtained.

### Example 10: Comparison of Reconstitution-Properties

The reconstitution times (time necessary for redissolving the voriconazole-cyclodextrin complexes) of complexes according to the present invention are compared with prior art complexes. The results are shown below in Table 4.

**Table 4**

| Test material | Characterization of reconstitution | Appearance of the reconstituted solution | Turbidity (τ) of the reconstituted solution recorded in time (stability test) determined in absorbance units (λ=410 nm) |
|---|---|---|---|
| Comparative Example 9 | complete dissolution was achieved after one minute by hand shaking | little bubbles | **>0.1** |
| VFEND™ | complete dissolution was achieved after one minute by hand shaking | clear solution | **0 h:** 0.02 ± 0.01<br>**24 h:** 0.02 ± 0.01 |
| Example 3 | complete dissolution was achieved **within approx. 20 seconds** by hand shaking | clear solution | **0 h:** 0.03 ± 0.01<br>**24 h:** 0.05 ± 0.01 |
| Example 1 | | | **0 h:** 0.04 ± 0.01<br>**24 h:** 0.06 ± 0.01 |
| Example 2 | | | **0 h:** 0.04 ± 0.01<br>**24 h:** 0.05 ± 0.01 |

Table 4 clearly shows that the voriconazole-cyclodextrin complexes of the present invention possess superior reconstitution properties.

**Example 11: Reduction of TAP-Content**

A solution of voriconazole and hydroxypropyl-β-cyclodextrin in 96 % ethanol was prepared. The coevaporate was isolated by evaporation of the solvent in a rotary evaporator. Subsequently, the TAP-content was determined. Different evaporation times (series 1) and evaporation temperatures (series 2) were used.

Series 1:

Settings: Evaporation time: 30 min
Evaporation temperature: 40 °C, 50 °C, 60 °C

Series 2:

Settings: Evaporation time: 30 min, 60 min, 120 min, 180 min Evaporation temperature: 40 °C

The results of series 1 are shown in Figure 4. The results of series 2 are shown in Figure 5.
As a conclusion it has been unexpectedly found that

a) with increasing temperature in the evaporation step the TAP-content is increasing. To get desirable low TAP contents, the temperature preferably should be between 30 °C and 65 °C, more preferably between 40 °C and 60 °C.
b) The time for evaporation has to be shortened in order to limit the TAP-content in the final product. Preferably a reaction time between 10 minutes and 120 minutes, more preferably between 20 minutes and 80 minutes, is used.

**Example 12: Reduction of Residual Ethanol Content in the Lyophilized Voriconazole-Cyclodextrin Complex**

A solution of voriconazole and hydroxypropyl-β-cyclodextrin in 96 % ethanol was prepared. A coevaporate was obtained by evaporation of the solvent in a rotary evaporator. The coevaporate was reconstituted with water for injection. The reconstituted solution lyophilized in situ (which means in vials). The following different amounts of reconstitution solution have been used and analyzed.

| Example | | 12a | 12b | 12c |
|---|---|---|---|---|
| Amount of water for injection: | | 5 ml / vial | 10 ml / vial | 15 ml / vial |

(continued)

| Example | | 12a | 12b | 12c |
|---|---|---|---|---|
| Appearance: | | clear, colourless | clear, colourless | clear, colourless |
| pH-value: | | 6.90 | 6.85 | 6.83 |
| Osmolality: | mosmol/kg | 1185 | 474 | 304 |
| Viscosity: | mPa*s | 5.624 | 2.261 | 1.683 |
| Density: | g/ml | 1.1063 | 1.0605 | 1.0362 |

The ethanol content of the lyophilistate according to Example 12a was significantly higher than 5000 ppm, the ethanol content according to Example 12b about 5000 ppm and the ethanol content according to Example 12c was significantly lower than 5000 ppm.
Hence, it was unexpectedly found that the ethanol content could be desirably lowered by using a specific amount of water for dissolving the coevaporate.

**Example 13: Reduction of Residual Ethanol Content in the Lyophilized Voriconazole-Cyclodextrin Complex**

A solution of voriconazole and hydroxypropyl-β-cyclodextrin in 96 % ethanol was prepared. A coevaporate was obtained by evaporation of the solvent in a rotary evaporator. The coevaporate was reconstituted with water for injection. The water was removed by evaporation and subsequently the resulting evaporate was again reconstituted with water for injection (= steps (iii-a), (iii-b) and (iii-c)).
The reconstituted solution was lyophilized in situ according to the conditions as described in Example 12b.
The ethanol content of the lyophilisate according to Example 13 was significantly lower than 5000 ppm.
Hence, it was unexpectedly found that the ethanol content could be desirably lowered by repeating step (iii) of the present invention, i.e. by carrying out sub-steps (iii-a), (iii-b) and (iii-c) of the present invention.

## Claims

**1.** Process for producing a coevaporate comprising

(a) voriconazole,
(b) cyclodextrin, and
(c) optionally an organic solvent,

said process comprising the steps of

(i) dissolving voriconazole and cyclodextrin in the organic solvent,
(ii) removing the solvent completely or partially.

**2.** Process according to claim 1, wherein the resulting coevaporate comprises

(a) 1 to 20 wt.%, preferably 5 to 15 wt.% voriconazole,
(b) 80 to 99 wt.%, preferably 85 to 95 wt.% cyclodextrin, and
(c) 0 to 10 wt.%, preferably 0,5 to 5 organic solvent.

**3.** Process according to claim 1 or 2, wherein the organic solvent has a relative permittivity from 3 to 40, measured at 20 °C.

**4.** Process according to any one of claims 1 to 3, wherein the solvent is an alcohol, preferably ethanol.

**5.** Process according to any one of claims 1 to 4, wherein in step (ii) the solvent is removed at a temperature between 40 °C and 60 °C.

**6.** Process for producing a voriconazole-cyclodextrin complex in solid form, said process comprises the steps of

(i) dissolving voriconazole and cyclodextrin in the organic solvent,
(ii) removing the solvent completely or partially in order to form a coevaporate,
(iii) dissolving the coevaporate in water, and
(iv) removing the water.

**7.** Process according to claim 6, wherein in step (iii) voriconazole is present in a concentration of 12 to 50 mg per ml water.

**8.** Process according to claims 6 or 7, wherein in step (iv) the water is removed by freeze-drying.

**9.** Process for producing sterile vials containing a voriconazole-cyclodextrin complex in solid form, said process comprises the steps of

(i) dissolving voriconazole and cyclodextrin in the organic solvent,
(ii) removing the solvent completely or partially in order to form a coevaporate,
(iii) dissolving the coevaporate in water,
(iii-2) subjecting the resulting aqueous solution to a sterilisation step,
(iii-3) filling the sterilized solution into vials,
(iv) removing the water by lyophilization, and
(v) sealing the vials.

**10.** Process of any of the preceding claims, wherein the cyclodextrin is hydroxypropylated beta-cyclodextrin.

**Patentansprüche**

**1.** Verfahren zur Herstellung eines Coevaporats umfassend

(a) Voriconazol
(b) Cyclodextrin und
(c) gegebenenfalls ein organisches Lösungsmittel.

wobei das Verfahren die folgenden Schritte umfasst

(i) Lösen des Voriconazols und Cyclodextrins in dem organischen Lösungsmittel,
(ii) Gänzliches oder teilweises Entfernen des Lösungsmittels.

**2.** Verfahren nach Anspruch 1, wobei das entstandene Coevaporat

(a) 1 bis 20 Gew.%, bevorzugt 5 bis 15 Gew.%, Voriconazol,
(b) 80 bis 99 Gew%, bevorzugt 85 bis 95 Gew%, Cyclodextrin und
(c) 1 bis 10 Gew.%, bevorzugt 0,5 bis 5 Gew%, organisches Lösungsmittel umfasst.

**3.** Verfahren nach Anspruch 1 oder 2, wobei das organische Lösungsmittel eine relative Permittivität von 3 bis 40, gemessen bei 20°C, aufweist

**4.** Verfahren nach einem der Ansprüche 1 bis 3, wobei das Lösungsmittel ein Alkohol, bevorzugt Ethanol, ist.

**5.** Verfahren nach einem der Ansprüche 1 bis 4, wobei in Schritt (ii) das Lösungsmittel bei einer Temperatur zwischen 40°C und 60°C entfernt wird.

**6.** Verfahren zur Herstellung eines Voriconazol-Cyclodextrin Komplexes in fester Form, wobei das Verfahren die folgenden Schritte umfasst

(i) Lösen von Voriconazol und Cyclodextrin in dem organischen Lösungsmittel,
(ii) vollständiges oder teilweises Entfernen des Lösungsmittels, um ein Coevaporat zu bilden,
(iii) Lösen des Coevaporats in Wasser und
(iv) Entfernen des Wassers.

7. Verfahren nach Anspruch 6, wobei in Schritt (iii) Voriconazol in einer Konzentration von 12 bis 50 mg pro ml Wasser vorliegt.

8. Verfahren nach einem der Ansprüche 6 oder 7, wobei in Schritt (iv) das Wasser durch Gefriertrocknung entfernt wird.

9. Verfahren zur Herstellung steriler Vials enthaltend einen Voriconazol-Cyclodextrin Komplex in fester Form, wobei das Verfahren die folgenden Schritte umfasst

   (i) Lösen von Voriconazol und Cyclodextrin in dem organischen Lösungsmittel,
   (ii) vollständiges oder teilweises Entfernen des Lösungsmittels, um ein Coevaporat zu bilden,
   (iii) Lösen des Coevaporats in Wasser,
   (iii-2) Durchführen eines Sterilisationsschrittes an der resultierenden wässrigen Lösung,
   (iii-3) Abfüllen der sterilisierten Lösung in Vials,
   (iv) Entfernen des Wassers durch Lyophilisierung und
   (v) Versiegeln des Vials.

10. Verfahren nach einem der vorstehenden Ansprüche, wobei das Cyclodextrin hydropropyliertes beta-Cyclodextrin ist.

**Revendications**

1. Procédé pour produire un coévaporat comprenant

   (a) du voriconazole,
   (b) de la cyclodextrine, et
   (c) facultativement un solvant organique,

   le dit procédé comprenant les étapes de

   (i) la dissolution de voriconazole et de cyclodextrine dans le solvant organique,
   (ii) l'enlèvement complet ou partiel du solvant.

2. Procédé selon la revendication 1, dans lequel le coévaporat résultant comprend

   (a) 1 à 20 % en poids, préférablement 5 à 15 % en poids de voriconazole,
   (b) 80 à 99 % en poids, préférablement 85 à 95 % en poids de cyclodextrine, et
   (c) 0 à 10 % en poids, préférablement 0,5 à 5 % en poids de solvant organique.

3. Procédé selon la revendication 1 ou 2, dans lequel le solvant organique a une permittivité rélative de 3 à 40, mesurée à 20°C.

4. Procédé selon l'une quelconque des revendications 1 à 3, dans lequel le solvant est un alcool, préférablement de l'éthanol.

5. Procédé selon l'une quelconque des revendications 1 à 4, dans lequel à l'étape (ii) le solvant est enlevé à une température entre 40°C et 60°C.

6. Procédé pour la fabrication d'un complexe de voriconazole-cyclodextrine en forme solide, le dit procédé comprenant les étapes de

   (i) la dissolution de voriconazole et de cyclodextrine dans le solvant organique,
   (ii) l'enlèvement complet ou partiel du solvant afin de former un coévaporat,
   (iii) la dissolution du coévaporat dans de l'eau, et
   (iv) l'enlèvement de l'eau.

7. Procédé selon la revendication 6, dans lequel à l'étape (iii) du voriconazole est présent en une concentration de 12 à 50 mg par ml d'eau.

**8.** Procédé selon la revendication 6 ou 7, dans lequel à l'étape (iv) l'eau est enlevé par lyophilisation.

**9.** Procédé pour la production de flacons stériles contenant un complexe de voriconazole-cyclodextrine en forme solide, le dit procédé comprenant les étapes de

(i) la dissolution de voriconazole et de cyclodextrine dans le solvant organique,
(ii) l'enlèvement complet ou partiel du solvant afin de former un coévaporat,
(iii) la dissolution du coévaporat dans de l'eau,
(iii-2) la soumission de la solution aqueuse obtenue à une étape de stérilisation,
(iii-3) le remplissage de la solution stérilisée en flacons,
(iv) l'enlèvement de l'eau par lyophilisation, et
(v) le scellage des flacons.

**10.** Procédé selon l'une quelconque des revendications précédentes, dans lequel la cyclodextrine est de la bêta-cyclodextrine hydroxypropylée.

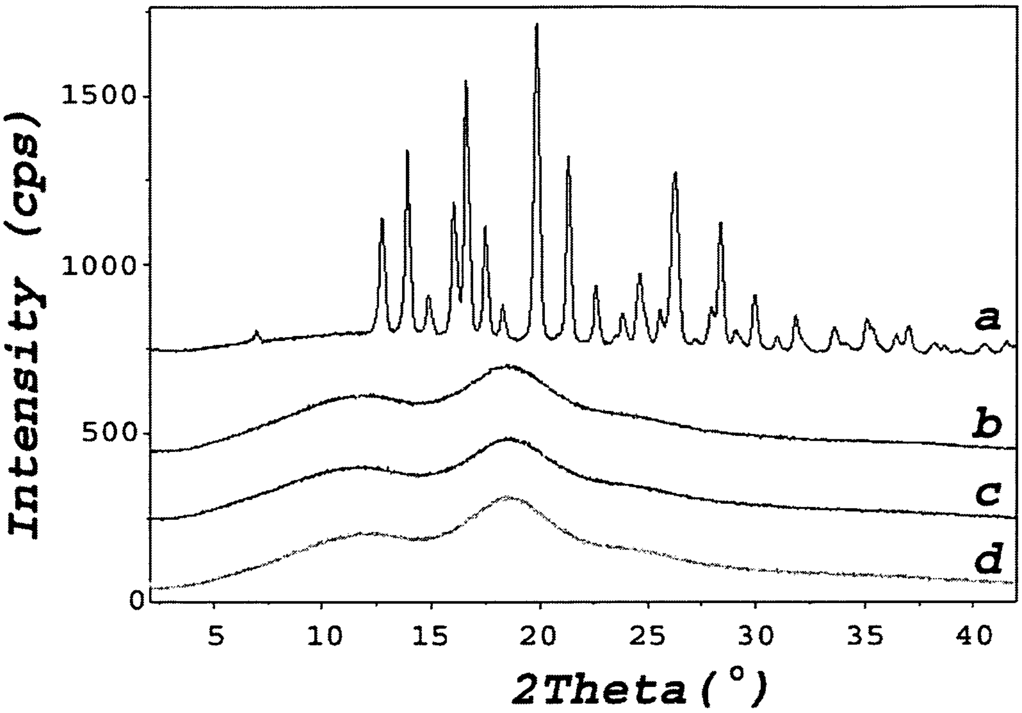


**Figure 1:** X-ray diffractograms:

a.): voriconazole

b.): voriconazole/HPBCD complex according to Example 2

c.): voriconazole/HPBCD complex according to Example 1

d.): voriconazole/HPBCD complex according to Example 3

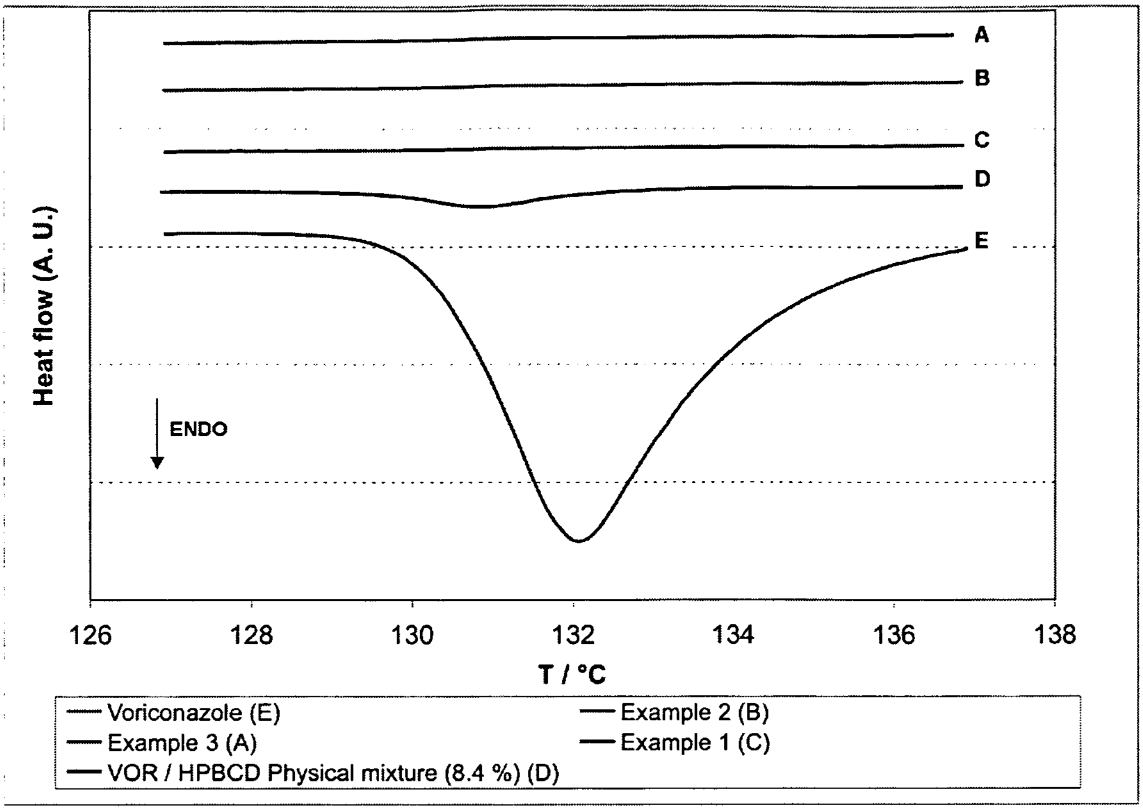


**Figure 2**: DSC of voriconazole preparations

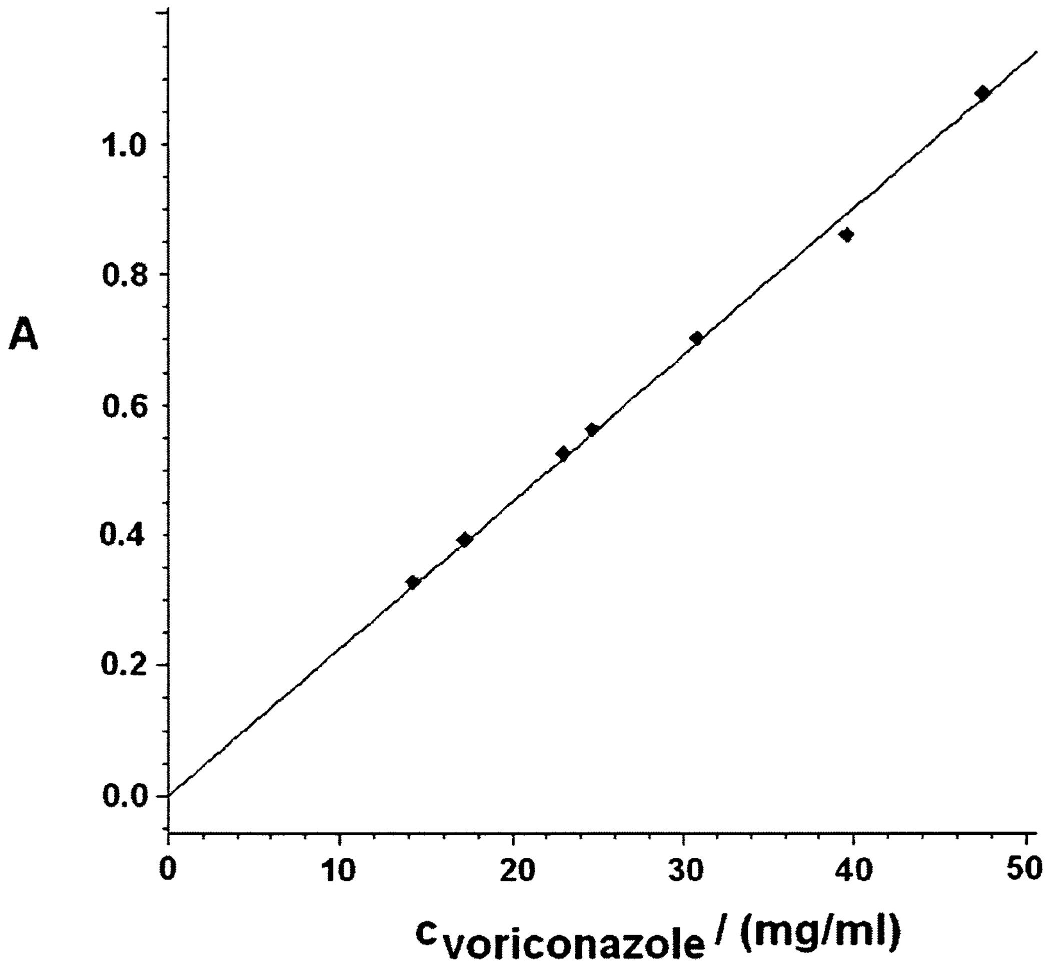


**Figure 3**: Absorbance A (l=256 nm) vs. concentration (mg/ml) plot for voriconazole with the fitted linear curve ($R^2$=0.99961)

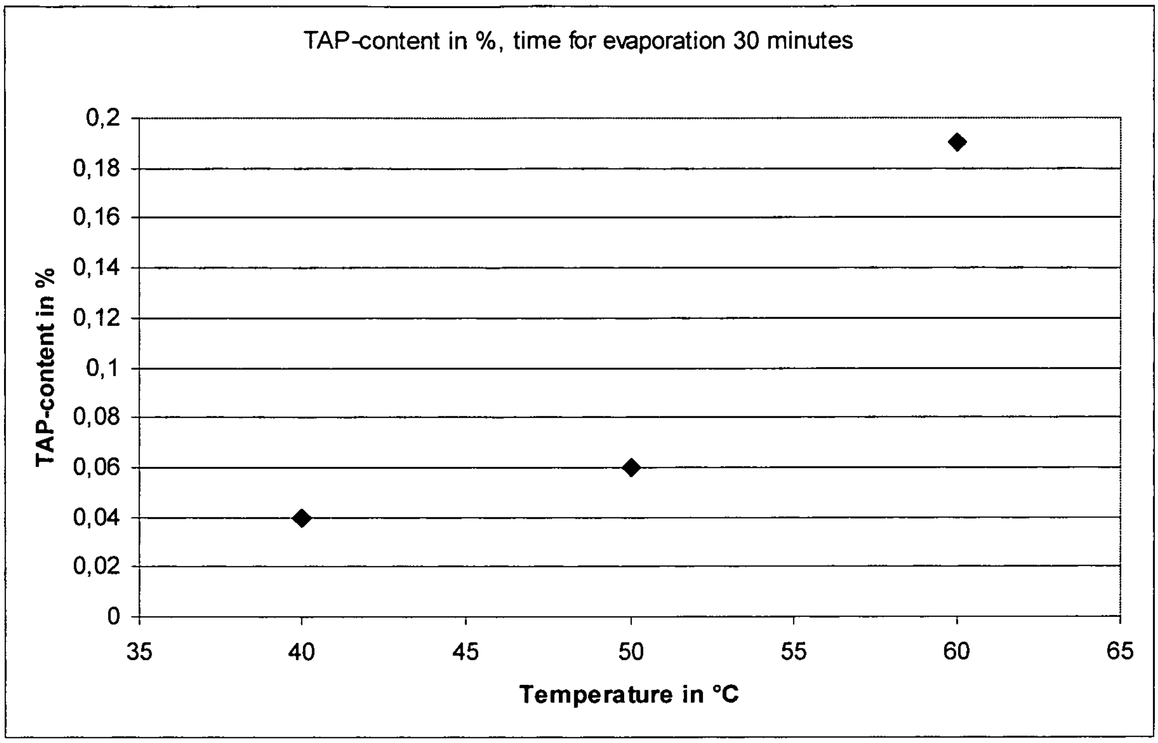


**Figure 4**

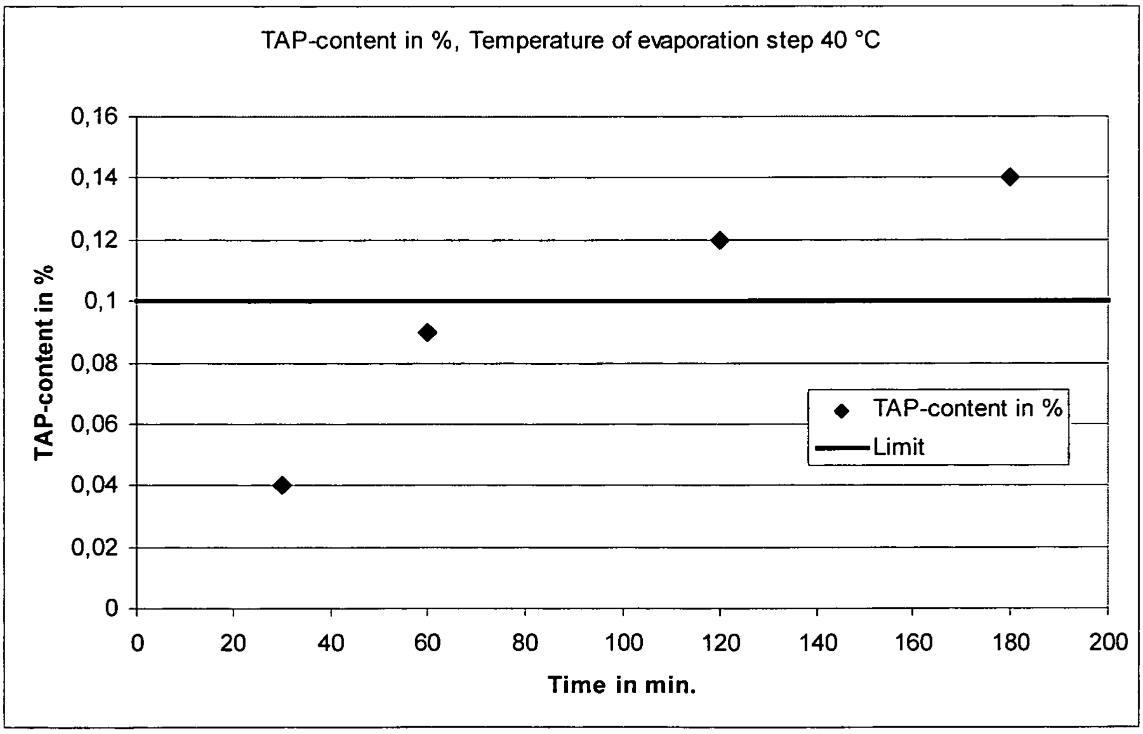


**Figure 5**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0440372 B1
- WO 9111172 A
- WO 9858677 A
- EP 2018866 A1
- EP 0440372 A
- CN 1813751 A
- EP 1001813 A

### Non-patent literature cited in the description

- Lexikon der Hilfsstoffe für Pharmazie, Kosmetik und angrenzende Gebiete
- Handbook of Pharmaceutical Excipients. American Pharmaceutical Association